# EUROPÄISCHE PATENTSCHRIFT

(11) **EP 2 090 269 B1**
(45) Veröffentlichungstag und Bekanntmachung des Hinweises auf die Patenterteilung: **23.02.2011**
(21) Anmeldenummer: 09150411.8
(22) Anmeldetag: 13.01.2009
(51) Int. Cl.: A61F 2/84, A61F 2/00, A61M 25/00, A61M 25/10, A61F 2/02

(54) **System zum Einbringen einer intraluminalen Endoprothese und Verfahren zur Herstellung eines derartigen Systems**
System for delivering an intraluminal endoprostheses and method for producing such a system
Système d'introduction d'une endoprothèse intraluminale et procédé de fabrication d'un tel système

(30) Priorität: 13.02.2008 DE 102008008926
(43) Veröffentlichungstag der Anmeldung: 19.08.2009
(73) Patentinhaber: Biotronik VI Patent AG, 6341 Baar (CH)
(72) Erfinder: Klocke, Björn, 8006, Zürich (CH); Martini, Claus, 8044, Zürich (CH); Fringes, Matthias, 91522, Ansbach (DE); Müller, Stefan, 8006 Zürich (CH)
(74) Vertreter: Lindner-Vogt, Karin L.

(56) Entgegenhaltungen:
- WO-A-2007/090385
- WO-A-2008/060877
- WO-A-2009/066330
- DE-U1- 20 321 514
- US-A- 5 102 402
- US-A- 5 226 887
- US-A- 5 478 319
- US-A- 6 013 055
- US-A1- 2005 177 130

## Beschreibung

Die vorliegende Erfindung bezieht sich auf ein System zum Einbringen einer intraluminalen Endoprothese, vorzugsweise eines Stents, in einen Körperhohlraum bestehend aus der intraluminalen Endoprothese und einem Katheter mit einem Ballon, wobei der Ballon in einem nicht dilatierten Zustand mindestens einen Flügel aufweist. Die Erfindung bezieht sich ferner auf ein Verfahren zur Herstellung eines derartigen Katheters.

Intraluminale Endoprothesen in Form von Stents werden derzeit weit verbreitet eingesetzt, da sie eine einfache und kostengünstige Behandlung ermöglichen. Sie weisen häufig ein rohrförmiges oder hohlzylinderförmiges Grundgitter auf, das an beiden Längsenden offen ist. Das Grundgitter einer derartigen Endoprothese wird mittels eines Katheters in den zu behandelnden Körperhohlraum eingesetzt und dilatiert oder freigesetzt (Nitinol-Stents). Nach Entfernen des Katheters dient die Endoprothese dazu, den Körperhohlraum zu stützen. Derartige Stents haben sich insbesondere zur Behandlung von Gefäßerkrankungen etabliert. Durch den Einsatz von Stents können verengte Bereiche in den Gefäßen so erweitert werden, dass ein Lumengewinn entsteht.

Katheter sind Röhrchen oder Schläuche verschiedenen Durchmessers, die in den jeweiligen zu behandelnden Körperhohlraum eingeführt werden können. Sogenannte Ballonkatheter werden vor allem in der Angioplastie zur Erweiterung oder Wiedereröffnung eines Gefäßes eingesetzt. Ein Führungsdraht wird zuerst in das zu behandelnde Gefäß eingeschoben und anschließend wird der Ballonkatheter, der aus mindestens einem Schlauch besteht, der in einem vorgegebenen Bereich entlang des Schlauchs einen nicht dilatierten, gefalteten Ballon aufweist, entlang des Führungsdrahts bis zu der zu behandelnden Stelle des Gefäßes vorgeschoben, so dass der Ballon im Bereich der zu behandelnden Stelle des Gefäßes, die z.B. eine Stenose auweist, platziert ist. Danach wird der Ballon dilatiert, d.h. entfaltet und aufgedehnt, so dass die zu behandelnde Stelle wiedereröffnet oder erweitert wird und der Strom der Körperflüssigkeit in dem Gefäß nicht mehr oder nicht mehr in dem zuvor vorhandenen Maße behindert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen.

Derartige Ballonkatheter können auch dazu verwendet werden, intraluminale Endoprothesen an eine zu behandelnde Stelle in einem Körperhohlraum einzubringen.

Intraluminale Endoprothesen werden heute häufig mit pharmazeutisch aktiven Substanzen wie Medikamenten versehen, die über einen bestimmten Zeitraum im Organismus freigesetzt werden.

Derartige pharmazeutisch aktive Substanzen können beispielsweise dazu dienen, Restenosen oder Agglomerationen zu vermeiden. Durch die Freisetzung von pharmazeutisch aktiven Substanzen, mit denen derartige intraluminale Endoprothesen versehen sind, ist es möglich, lediglich eine lokale Behandlung, d.h. eine Elution eines Wirkstoffs im Wesentlichen nur in das die intraluminale Endoprothese umgebende Gewebe, vorzunehmen. Dieser Vorgang wird auch als "Local Drug Delivery" (LDD) bezeichnet. Der Behandlungsort, an dem der Wirkstoff seine pharmakologische Wirkung entfalten soll, grenzt somit unmittelbar an den Ort der Implantation der intraluminalen Endoprothese.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe der Taxole oder Taxane, hier vorzugsweise Paclitaxel oder Sirolimus, bestehen.

Derzeit werden auch intraluminale Endoprothesen eingesetzt, die aus einem Material bestehen, das der Biodegradation unterliegt. Hierbei werden unter Biodegradation hydrolytische, enzymatische oder andere stoffwechsel-bedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Derartige biodegradierbare Materialien können aus Polymeren oder Metallen realisiert werden. Im Zusammenhang mit Stents ist auch die Abkürzung AMS (Absorbierbarer Metall Stent) gebräuchlich. Derartige Stents enthalten ein biodegradierbares Metall, vorzugsweise Magnesium, Eisen, Zink, Wolfram und/oder eine Legierung der genannten Metalle.

Bei intraluminalen Endoprothesen, die aus einem biodegradierbaren Material bestehen und außerdem mit einer pharmazeutisch aktiven Substanz versehen sind, entsteht häufig das Problem, dass die pharmazeutisch aktiven Substanzen nicht richtig an dem Grundgitter der Endoprothese haften oder nicht in der gewünschten Weise funktionieren, da sich bei der Biodegradation der Endoprothese der pH-Wert der Umgebung verändern und/oder die Endoprothese unkontrolliert korrodieren kann und dabei stark unterwandert wird. Die Abgabe der pharmazeutisch aktiven Substanz erfolgt somit unkontrolliert bzw. nicht in der gewünschten Weise oder dem gewünschten Zeitraum.

In der Druckschrift DE 691 19 753 T2 wird ein Ballonkatheter beschrieben, der einen Katheterkörper und einen Ballon aufweist, der entlang der Länge des Katheterkörpers angeordnet ist. Der Ballon ist ferner mit einer Einrichtung zu seinem Aufblasen und Ablassen von außen und einer Einrichtung zum Zuführen eines Medikaments oder einer Kombination von Medikamenten zur Behandlung oder Diagnose innerhalb eines Körperhohlorgans versehen, wenn der Katheter in diesem positioniert und aufgeblasen wird. Die Zuführeinrichtung weist Mikrokapseln an der Außenseite des Ballons auf, wobei die Mikrokapseln in Falzen am Ballon, wie sie ausgebildet sind, wenn der Ballon geschrumpft ist, an ihm festgehalten werden. Hierbei sind die Mikrokapseln derart gestaltet, dass sie zerreißbar oder abbaubar sind. Sie öffnen sich, wenn sie an den Hohlorganwänden zurückgelassen werden. Die Kapseln können auch aufgrund des Anlegens von Ultraschallwellen zerreißen.

Der Nachteil des in der oben angegebenen Druckschrift beschriebenen Katheters besteht darin, dass zur Realisierung der Medikamentenabgabe mikroverkapselte Medikamente verwendet werden müssen, die in ihrer Herstellung teuer sind. Eine Mikroverkapselung ist zudem nicht für jedes in Frage kommende Medikament herstellbar. Außerdem weist ein Ballon eines derartigen, mit Mikrokapseln versehenen Katheters einen vergleichsweise großen Durchmesser auf, dessen Profil in der Praxis nicht verwendbar ist und den Katheter steif und unflexibel macht. Zudem kann das Medikament nicht nur an der zu behandelnden Stelle sondern auch beim Einführen oder der Dilatation des Ballons abgerieben werden. Hierdurch werden die mit der Behandlung verbundenen Nebenwirkungen erhöht.

Aus dem Dokument DE 203 21 514 U1 geht ein Kathetersystem hervor, das einen Katheter, einen mit Falten versehenen Ballon und einen auf den Ballon fest angeordneten Stent aufweist. Medikamente können durch ein Tauchenverfahren zwischen die engen Falten des Ballons dringen, wobei das Verfahren zu keiner Verklebung der Faltung führt.

Aus dem Dokument WO 2007/090385 ist ein weiterer mit Falten versehener Katheterballon bekannt, dessen Falten in einem nicht expandierten Zustand mit einer wirkstoffhaltenden Zusammensetzung beschichtet oder befüllt werden können. Es wird kein Polymer als Wirkstoffträger eingesetzt, um eine eventuelle teilweise Verklebung der Falten zu vermeiden.

Die Aufgabe der vorliegenden Erfindung besteht somit darin, ein System zu schaffen, das die Freisetzung einer pharmazeutisch aktiven Substanz beim Einbringen einer intraluminalen Endoprothese auf den Ort beschränkt, an dem die intraluminale Endoprothese eingesetzt wird, ohne dass die pharmazeutisch aktive Substanz vorher auf dem Weg durch das Gefäßsystem abgewischt wird. Die Aufgabe besteht ferner darin, ein Verfahren zur Herstellung eines derartigen Systems anzugeben, das einfach und kostengünstig ist.

Die oben angegebene Aufgabe wird durch ein System gelöst, bei dem im nicht dilatierten Zustand mindestens eine pharmazeutisch aktive Substanz zumindest teilweise unter dem mindestens einen Flügel des Ballons angeordnet ist, wobei die intraluminale Endoprothese auf dem gefalteten Ballon des Katheters derart fest angeordnet ist, dass sie diesen mindestens teilweise umgibt, und wobei der mindestens eine Flügel des Ballons mittels der mindestens einen pharmazeutisch aktiven Substanz verklebt ist.

Hierbei beinhaltet der "nicht dilatierte Zustand" alle Zustände des Ballons des Katheters, in dem dieser nicht vollständig entfaltet vorliegt, d.h. noch mindestens einen Flügel an dem Ballon mindestens teilweise vorhanden ist, auch wenn dieser bereits teilweise aufgedehnt ist. Ebenso werden Zustände mit einbezogen, bei denen der Ballon deflatiert ist, d.h. bereits einmal dilatiert war und anschließend wieder zusammengefaltet wurde. Mit dem "nicht dilatierten Zustand" werden aber insbesondere Zustände angesprochen, in denen der Ballon nach dem Falten und Prägen vollständig gefaltet vorliegt bzw. nach dem Deflatieren wieder fast vollständig eingefaltet ist.

Außerdem bedeutet "unter dem mindestens einen Flügel des Ballons angeordnet", dass die mindestens eine pharmazeutisch aktive Substanz innerhalb der Falte des Ballons angeordnet ist, d.h. auf oder in den Flächen des Flügels angeordnet ist, die nach dem Falten übereinander liegen. Die unter dem mindestens einen Flügel angeordnete pharmazeutisch aktive Substanz wird somit von dem außen liegenden Teil des jeweiligen Flügels abgedeckt.

Weiterhin wird durch die Angabe, dass die Endoprothese den gefalteten Ballon mindestens teilweise umgibt, ausgedrückt, dass die Endoprothese außen auf den nach der Faltung außen liegenden Flächen des Ballons angeordnet ist. Die Endoprothese bedeckt diese außen liegenden Flächen dabei mindestens teilweise. Hierbei ist die Anordnung der Endoprothese auf dem Ballon nicht als dauerhaft fest anzusehen. Die Endoprothese ist bis zum Beginn der Ballondilatation fest auf dem Ballon angeordnet, also auch bei der Lagerung und beim Einführen in den menschlichen oder tierischen Körper. Nach dem Erreichen des maximal dilatierten Zustandes, wenn der Ballon wieder entleert wird, verbleibt die Endoprothese im Körperhohlraum, während der Katheter aus diesem entfernt wird.

Durch das oben angegebene System kann die nicht mikroverkapselt vorliegende pharmazeutisch aktive Substanz direkt dort, wo die intraluminale Endoprothese in den Körperhohlraum implantiert wird, an die umgebende Körperflüssigkeit und das Gewebe abgegeben werden. Vor dem Abgeben wird die pharmazeutisch aktive Substanz vor Abreibung und Auswaschung geschützt. Zudem ist durch die feste Anordnung der intraluminalen Endoprothese auf dem Ballon gewährleistet, dass das System aus intraluminaler Endoprothese und Ballon beim Einbringen des Systems in den Körperhohlraum ein insgesamt sehr kleines Volumen einnimmt, so dass es flexibel und einfach handhabbar ist. Es ist außerdem nicht notwendig, die pharmazeutisch aktive Substanz in einer Mikroverkapselung bereitzustellen. Zudem kann die Freigabe der mindestens einen pharmazeutisch aktiven Substanz aufgrund des Fehlens der Mikroverkapselung sofort nach der Dilatation des Ballons zusammen mit der intraluminalen Endoprothese erfolgen, so dass sich eine schnelle Wirksamkeit der pharmazeutisch aktiven Substanz und eine Wirksamkeit genau am Ort der Behandlung ergibt.

In einem besonders bevorzugten Ausführungsbeispiel ist bei einem erfindungsgemäßen Katheter die intraluminale Endoprothese auf den Ballon aufgecrimpt. Dies ist eine sehr einfache und kostengünstige Anbringungsweise der intraluminalen Endoprothese auf dem Ballon.

Ferner ist es von Vorteil, wenn die intraluminale Endoprothese als biodegradierbarer Stent, vorzugsweise als absorbierbarer Metallstent, ausgebildet ist. Ein derartiger Stent ist nach Erfüllung seiner Behandlungsfunktion nicht länger im Gewebe des behandelten Körperhohlraums vorhanden (löst sich nahezu vollständig auf) und verursacht so weniger Nebenwirkungen.

In einem weiteren bevorzugten Ausführungsbeispiel ist die mindestens eine pharmazeutisch aktive Substanz auf oder in einem Träger oder mehreren Trägern angeordnet, die unter einem Flügel oder mehreren Flügeln des Ballons angeordnet sind. Hierdurch ist es in besonders einfacher Weise möglich, einen bereits einmal verwendeten Katheter wieder zum Einbringen einer intraluminalen Endoprothese unter Abgabe einer pharmazeutisch aktiven Substanz einzusetzen, da ein oder mehrere Träger leicht unter der oder den Flügeln angeordnet werden können.

Die Flügel des Ballons sind mittels der mindestens einen pharmazeutisch aktiven Substanz verktebt. Hierdurch verstärkt sich der Effekt, dass die pharmazeutisch aktive Substanz aus den Falten nicht unkontrolliert entweicht und die Haftabzugskraft erhöht wird.

Eine besonders gute Abdeckung des mit der pharmazeutisch aktiven Substanz versehenen Ballons im nicht dilatierten Zustand wird dann erreicht, wenn in einem bevorzugten Ausführungsbeispiel die intraluminale Endoprothese die darunter liegenden äußeren Flächen des gefalteten Ballons vollständig bedeckt. Hierbei werden mit "äußeren Flächen" die Außenflächen oder Oberflächenbereiche des gefalteten Ballons gemeint, die nach dem Falten und Prägen außen liegen. Die unterhalb des Flügels oder der Flügel angeordneten, übereinander liegenden Außenflächen oder Oberflächenbereiche des Ballons gehören nicht zu den äußeren Flächen.

Besonders kostengünstig herstellbar ist der Katheter des Systems, wenn die mindestens eine pharmazeutisch aktive Substanz mittels Tauchen, Sprühen, Bestreichen oder Pressen auf dem Ballon aufgebracht ist.

In einem besonders bevorzugten Ausführungsbeispiel ist die intraluminale Endoprothese luminal mit einer leicht lösbaren Beschichtung versehen, vorzugsweise mit einer oder mehreren der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle. Eine derartige Schicht wird nach der Implantation durch die Körperflüssigkeit leicht abgewaschen, so dass keine Wirkstoffreste an der luminalen Seite der intraluminalen Endoprothese verbleiben und somit die Endothelialisierung nicht behindert wird.

In einem besonders bevorzugten Ausführungsbeispiel weist der Ballon des erfindungsgemäßen Systems mindestens ein im Wesentlichen in Längsrichtung verlaufendes Faltelement auf, das beim Falten des Ballons in einem der in Längsrichtung verlaufenden Bereiche des oder der Flügel mit einem Minimum im Biegeradius angeordnet ist. Das bedeutet, dass der Ballon entlang des Faltelements gefaltet wird. Durch ein derartiges Faltelement wird die richtige und reproduzierbare Anordnung der Flügel am Ballon erleichtert.

Hierbei ist unter der Längsrichtung die Richtung der Achse des Katheters zu verstehen. Mit dem "im Wesentlichen in Längsrichtung verlaufenden Faltelement" ist ein Faltelement gemeint, das überwiegend in Längsrichtung verläuft, d.h. auch schräg oder spiralig in Längsrichtung, also mit einer Komponente in eine Richtung senkrecht zur Längsrichtung, verlaufen kann. Falten oder Faltlinien sind Bereiche der Ballonmembran, in denen diese ein Minimum im Biegeradius aufweisen. Diese Falten entstehen, wenn die Ballonmembran außen (am äußeren Flügelende) oder innen (an der Verbindungsstelle benachbarter Flügel) überdehnt wird. Unter dem Begriff "Falten" im Zusammenhang mit "Falten des Ballons" wird sowohl das erstmalige Falten des Ballons bei der Herstellung des Katheters als auch das Zurückfalten (Rewrap) beim Deflatieren verstanden.

Hierbei ist es besonders bevorzugt, wenn der Ballon entlang des mindestens einen Faltelements einen Bereich des Ballons ausbildet, der verglichen mit den übrigen Bereichen des Ballons eine veränderte Steifigkeit, vorzugsweise eine niedrigere Steifigkeit, aufweist.

Dadurch wird die Faltung entlang der durch das Faltelement vorgegebenen Faltlinie des Ballons erleichtert, da die Ballonmembran immer entlang der schwächsten Stelle einknickt, sobald der Ballon entleert wird.

Eine bevorzugte und einfache Möglichkeit, Steifigkeitsunterschiede in die Ballonmembran zu integrieren, besteht darin, dass der Ballon im Bereich des mindestens einen Faltelements Vertiefungen oder Erhebungen oder mindestens einen Wandstärkensprung aufweist.

Alternativ oder zusätzlich zu den oben genannten Möglichkeiten, Steifigkeitsunterschiede in den Ballon zu integrieren, besteht außerdem die vorteilhafte Möglichkeit, dass das mindestens eine Faltelement einen Bereich des Ballons ausbildet, der eine von den übrigen Bereichen des Ballons verschiedene Materialzusammensetzung aufweist. Diese Bereiche (mit der größten Ausdehnung in Längsrichtung des Ballons verlaufend und bspw. mit einem quadratischen, kreisförmigen, ellipsenförmigen oder rechteckigen Querschnitt aufweisend) sind bspw. stegförmige ausgebildet, wobei ein solcher Steg auf der Oberfläche des Ballons oder eingebettet in das Volumen des Ballons vorgesehen werden können. Beispielsweise können bei einem Ballonmaterial aus PEBAX in Längsrichtung auf der Außenseite entlang des Ballons verlaufende Bereiche mit dem Material PA12 eingebracht werden, die beim Falten unter stärkerem Zug als das Ballonmaterial stehen. Beim Falten des Ballons werden sich diese Bereiche somit in den Minima des Biegeradius anordnen, bei denen ein Flügel des Ballons an den benachbarten Flügel angrenzt. Bei der Verwendung von PA12 als Ballonmaterial können umgekehrt in Längsrichtung auf der Innenseite entlang des Ballons verlaufende Bereiche des Materials PEBAX eingebracht werden. Diese Bereiche werden sich beim Falten des Ballons an der Spitze eines Flügels anordnen, da diese Bereiche unter geringerer Zugspannung als das übrige Ballonmaterial stehen. Beide Varianten können auch kombiniert werden. Weitere Materialkombinationen können außerdem die Materialien PA11 und PVC nutzen. In einem weiteren Ausführungsbeispiel, bei dem ebenfalls die genannten Materialien verwendet werden können, wird das nicht zu den Faltelementen gehörende Material des Ballons mit einer Verstärkungsschicht versehen werden, die Bereiche erhöhter Wandstärke erzeugt. Die Verstärkungsschicht kann als Zwischenschicht in das Material des Ballons eingebettet sein.

In einem weiteren bevorzugten Ausführungsbeispiel weist das mindestens eine Faltelement Unterbrechungen auf, die für eine größere Stabilität der Faltlinien sorgen.

Ebenfalls bevorzugt ist ein System, bei dem das Faltelement an dem Ballon in einem fest vorgegebenen Winkel zur Ballonachse verläuft. Dies bedeutet auch, dass der jeweilige Flügel in einem Winkel zur Ballonachse erzeugt wird. Hierbei verläuft das mindestens eine Faltelement auf oder in der Fläche des Ballons um den Ballon herum und nicht lediglich parallel zur Ballonachse. Hierdurch wird ein gleichmäßiges Biegemoment des gefalteten Ballons um seine Längsachse erzeugt. Bei einer von außen aufgebrachten Biegung des Systems heben sich Zugkräfte und Kompressionen weitgehend auf, Dehnungen und Streckungen kompensieren sich. Der erfindungsgemäße Katheter zeichnet sich durch eine gleichmäßige Trackability aufgrund des winkelunabhängigen Biegemoments des gefalteten Ballons aus. Ferner stellen sich die umlaufenden Flügel in engen Kurven nicht mehr auf, da sich die herrschenden Kräfte, wie oben beschrieben, ausgleichen und der steife Flügel nicht mehr senkrecht zur Beanspruchungsrichtung steht und damit Deformationen an ihrer Flanke besser abbauen kann.

Bei einem weiteren Ausführungsbeispiel eines erfindungsgemäßen Katheters werden die Faltelemente des Ballons durch Längsstreben ausgebildet, die ein Gerüst bilden, welches an der Innenseite und/oder an der Außenseite des Ballons angeordnet ist und das den Ballon an definierten Stellen stützt. Dieses Gerüst, das im Folgenden auch als Self-X-Gerüst bezeichnet wird, hat den Vorteil, dass es nachgerüstet werden kann. Zudem lassen sich mit dem Gerüst je nach Wahl des Gerüstmaterials sehr große Spannungen beim Falten des Ballons aufnehmen. Die Längsstreben können auch spiralig verlaufen. Als Gerüstmaterialien können beispielsweise Nitinol oder Thermoplaste verwendet werden.

In einem weiteren Ausführungsbeispiel enthält die pharmazeutisch aktive Substanz Taxole und/oder Taxane, besonders bevorzugt Paclitaxel und/oder Sirolimus, und/oder vorzugsweise mindestens einen hyperplastischen Wirkstoff mit einem Verteilungskoeffizienten zwischen Butanol und Wasser von ≥ 0,5. Hierbei dient der ggf. in der pharmazeutisch aktiven Substanz enthaltene hyperplastische Wirkstoff zur örtlich begrenzten Behandlung potentiell hyperproliferativen Gewebes. Als antihyperplastischer Wirkstoff können beispielsweise ein Cytostatikum, ein Kortikoid, ein Prostacyclin, ein Antioxidans, ein Mittel zur Hemmung der Zellproliferation oder ein Immunsuppressivum verwendet werden.

Die oben angegebene Aufgabenstellung wird außerdem gelöst durch ein Verfahren zur Herstellung eines derartigen Systems bestehend aus einem Katheter mit einem Ballon und einer intraluminalen Endoprothese, wobei zunächst die mindestens eine pharmazeutisch aktive Substanz auf oder in die äußere Oberfläche des Ballons des Katheters, vorzugsweise mittels Tauchen, Sprühen, Bestreichen oder Pressen, auf- oder eingebracht wird, anschließend der Ballon mit mindestens einem Flügel versehen, dann dieser mindestens eine Flügel eng angelegt (d.h. der Ballon auf einen möglichst kleinen Durchmesser gewickelt und in dieser Position thermisch fixiert) und danach die intraluminale Endoprothese auf dem gefalteten Ballon derart fest angeordnet wird, dass sie diesen mindestens teilweise umgibt.

Das erfindungsgemäße Herstellungsverfahren des Systems ist kostengünstig und leicht durchführbar und ergibt ein System, das die gewünschte lokale Behandlung des Körperhohlraums ermöglicht.

Weiter vereinfacht wird das Herstellungsverfahren, wenn die intraluminale Endoprothese mittels Aufcrimpen auf dem Ballon angeordnet wird.

In einem weiteren bevorzugten Ausführungsbeispiel werden die distalen und proximalen Enden des Ballons beim Aufbringen der mindestens einen pharmazeutisch aktiven Substanz ausgespart, vorzugsweise durch Abdecken während des Aufbringens der Substanz. Eine unkontrollierte Freigabe der mindestens einen pharmazeutisch aktiven Substanz aus den Bereichen des proximalen oder distalen Endes des Ballons, die möglicherweise nicht vollständig von der Endoprothese umgeben sind, wird hierdurch verhindert.

Aus dem gleichen Grund ist ein Verfahren bevorzugt, bei dem die überschüssige mindestens eine pharmazeutische aktive Substanz auf den nach dem Falten außen liegenden äußeren Flächen oder Oberflächen des Ballons nach dem Faltschritt entfernt wird, vorzugsweise durch Wegwischen. Hierdurch wird gewährleistet, dass die mindestens eine pharmazeutisch aktive Substanz lediglich unter den Flügeln angeordnet wird. Zum Wegwischen der pharmazeutisch aktiven Substanz kann beispielsweise poröses Zellpapier, Schwamm oder Ähnliches, gegebenenfalls getränkt mit einem Lösungsmittel, verwendet werden.

Als besonders vorteilhaft hat sich außerdem erwiesen, dass die mindestens eine pharmazeutisch aktive Substanz vor der Anordnung der intraluminalen Endoprothese auf dem Ballon ausgehärtet oder polymerisiert wird. Eine Aushärtung kann auch mit Hilfe eines Polymers oder eines Lösungsmittels auf der Ballonoberfläche erfolgen. Die pharmazeutisch aktive Substanz haftet hierdurch besonders gut an der Oberfläche des Ballons. Das Aushärten oder Polymerisieren wird insbesondere durch eine UV-Bestrahlung, eine Bestrahlung mittels Betastrahlen und/oder eine thermische Behandlung realisiert.

Die mindestens eine pharmazeutisch aktive Substanz wird vorzugsweise zusammen mit einem Träger oder Carrier (z.B. einem Polymer und/oder einem Lösungsmittel) aufgetragen. Im Fall der Verwendung eines Lösungsmittel verdampft dieses beim und/oder nach dem Auftragen. Das Austreiben des Lösungsmittels bei gleichzeitigem Aushärten des Polymer-Carriers wird auch als Curen bezeichnet. Als Lösungsmittel kommen, abhängig von der pharmazeutisch aktiven Substanz, vorzugsweise folgende Stoffe in Betracht: DMSO, Aceton, Ether (Di-ethylether), Methanol, Isopropanol, Ester so wie weitere geeignete Alkohole. Beim Einsatz von Polymeren oder einer polymerähnlichen Substanz als Träger und Aushärthilfe, muss darauf geachtet werden, dass die Polymere oder die polymerähnliche Substanz leicht löslich sind oder das Medikament schnell abgeben. Unter diesem Gesichtspunkt eignen sich besonders Hyaloronsäure, P4HB, Polyvinylpyrolidon, Liposome, Nanopartikel, Seidenproteine und Cyclodextrine.

Zusätzlich zur pharmazeutisch aktiven Substanz und ggf. einem ebenfalls aufgetragenen Trägers können Kontrastmittel (z.B. Ultravist) und/oder anorganische Salze (z.B. Natriumchlorid, Natriumcarbonat) und/oder organische Salze (z.B. Salze der Essigsäure, Zitronensäure, Weinsäure) sowie weitere feste Zusatzstoffe aufgetragen werden. Diese dienen zur Verbesserung der mechanischen Haftung auf der Oberfläche des Ballons und/oder zur Verbesserung der Abgabe der pharmazeutisch aktiven Substanz an die Gefäßwand und/oder zur Verbesserung der Aufnahmefähigkeit der Gefäßwand für die pharmazeutisch aktive Substanz.

Bei der Verwendung eines Trägers für die pharmazeutisch aktive Substanz, das ein auszutreibendes Lösungsmittel enthält, wird das erfindungsgemäße Verfahren vorzugsweise derart abgewandelt, dass nach dem Auftragen des Trägers mit Lösungsmittel und pharmazeutisch aktiver Substanz der Ballon gefaltet wird und das nach dem Auftragen außen liegende, überschüssige Material entfernt wird. Anschließend wird der Ballon aufgeblasen (dilatiert) und das Lösungsmittel, beispielsweise durch Wärmebehandlung, ausgetrieben. Danach ist die pharmazeutisch aktive Substanz, die ggf. in dem Träger weitere, oben beschriebene Zusatzstoffe enthält, fest auf der Oberfläche des Ballons angeordnet. Anschließend kann der Ballon wieder gefaltet werden und die pharmazeutisch aktive Substanz ist unter den Flügeln des Ballons angeordnet, wobei hierfür der Ballon, vorzugsweise durch Verwendung eines selbstfaltenden Ballons, an den gleichen Stellen wie bei der ersten Faltung zusammen gefaltet werden muss, so dass die mindestens eine pharmazeutisch aktive Substanz zuverlässig unter den Flügeln des Ballons liegt.

Zur Verbesserung der Gleichmäßigkeit der aufgebrachten pharmazeutisch aktiven Substanz wird diese in einem vorteilhaften Ausführungsbeispiel des erfindungsgemäßen Verfahrens dann auf dem Ballon aufgebracht, wenn sich der Ballon im dilatierten Zustand befindet.

Wie bereits oben erläutert, ist es von Vorteil, wenn die intraluminale Endoprothese vor der Anordnung der intraluminalen Endoprothese auf dem Ballon luminal mit einer leicht abwaschbaren Beschichtung, vorzugsweise mit einer oder mehrerer der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle, versehen wird.

Oben wurden bereits die Vorteile einer Anordnung von mindestens einem Faltelement an dem Ballon erläutert. Ein mit einem Faltelement versehener Ballon ist reproduzierbar selbst faltend. Ein vorteilhaftes Herstellungsverfahren eines erfindungsgemäßen Systems beinhaltet die Herstellung des Ballons vor der Verbindung mit den übrigen Teilen des Katheters mittels Blasformen, wobei die Blasform dort, wo das mindestens eine Faltelement entstehen soll, mit einer Vertiefung oder einer Erhebung, beispielsweise in Form von runden oder länglichen Noppen oder unterbrochenen Rillen, versehen ist. Alternativ kann der Ballon mittels Spritzblasen hergestellt werden, wobei im Bereich des mindestens einen Faltelements mindestens ein definierter Wandstärkesprung erzeugt wird. Dieser Wandstärkesprung kann z.B. schon im Vorformling durch ein entsprechendes Extrusionswerkzeug oder Spritzgießwerkzeug erzeugt werden. Sowohl durch die Vertiefungen und Erhebungen der Blasform als auch durch den mindestens einen Wandstärkesprung beim Spritzblasen werden Steifigkeitsunterschiede im Ballon erzeugt.

Wie oben bereits erläutert, ist es außerdem vorteilhaft, den Ballon an seiner Innenseite und/oder seiner Außenseite mit einem Längsstreben als Faltelemente enthaltenden Gerüst verbunden wird, welches den Ballon in bestimmten Bereichen stützt. Das Stützgerüst kann aus einem hochelastischen Material, das auch im maximal dilatierten Zustand (Rated Burst Pressure) immer noch an der Balloninnenwand anliegen muss, hergestellt werden. Wird der Balloninnendruck gesenkt, dann wird der Ballon nur noch im Bereich des Stützgerüsts offen gehalten und kollabiert in den nicht gestützten Bereichen zwischen den Längsstreben. So kann der Verlauf der Falten sehr genau gesteuert werden. Ein derartiges Gerüst könnte bspw. aus zwei spiralförmig verlaufenden Nitinoldrähten bestehen, die in den Ballonhälsen an Ringen verschweißt sind.

Das mindestens eine Faltelement kann auch derart vorteilhaft erzeugt werden, dass der Ballon im Bereich der mindestens einen Faltlinie, vorzugsweise vor dem Aufbringen der mindestens einen pharmazeutisch aktiven Substanz, lokal thermisch, beispielsweise mittels eines Lasers, und/oder mittels eines Lösungsmittels behandelt wird. Außerdem kann der Ballon, ebenfalls vorzugsweise vor dem Aufbringen der mindestens einen pharmazeutisch aktiven Substanz, im Bereich außerhalb der mindestens einen Faltlinie mit einem weiteren verstärkenden Material versehen werden.

Wie oben bereits erläutert ist es von Vorteil, wenn das mindestens eine Faltelement derart vorgesehen wird, dass es unter einem fest vorgegebenen Winkel zur Ballonachse auf der Oberfläche des Ballons oder in der Ballonfläche verläuft.

In einem weiteren Ausführungsbeispiel ist der Innenschaft und der Außenschaft des Katheters vor dem Verbinden miteinander verdreht und/oder verschoben werden, wobei der Ballon vor dem Verdrehen und/oder Verschieben bereits mit Innenschaft und Außenschaft verbunden ist. Der verdrehte und/oder verschobene Zustand ist bei diesem Ausführungsbeispiel der Normalzustand des Instruments. Beim Dilatieren speichern Innenschaft und Außenschaft die Torsionsenergie und begünstigen somit das spiralige Rückfalten des Ballons beim Deflatieren.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: einen Querschnitt durch ein erstes Ausführungsbeispiel eines erfindungs- gemäßen Systems,
- Fig. 2: einen Querschnitt durch ein zweites Ausführungsbeispiel eines erfindungs- gemäßen Systems und
- Fig. 3: einen Querschnitt durch ein drittes Ausführungsbeispiel eines erfindungs- gemäßen Systems.

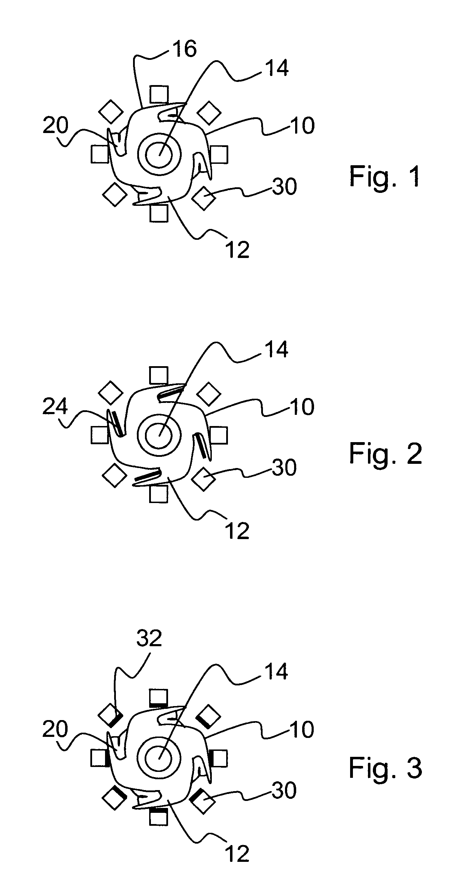

Das in Figur 1 dargestellte erste Ausführungsbeispiel eines erfindungsgemäßen Systems zeigt den Katheter im nicht dilatierten Zustand. Der Katheter weist einen Ballon 10, der in vier Flügel 12 gelegt ist, sowie einen innerhalb des Ballons angeordneten Innenschaft 14 auf. Unter jedem der Flügel 12 ist eine pharmazeutisch aktive Substanz 20 auf der Oberfläche des Ballons 10 angeordnet. In weiteren Ausführungsbeispielen des erfindungsgemäßen Systems können an der gleichen Stelle auch mehrere pharmazeutisch aktive Substanzen vorgesehen sein.

Die intraluminale Endoprothese in Form eines Stents 30 ist mit seinem Grundgitter auf der Oberfläche des gefalteten Ballons 10 fest angeordnet. Bei der Darstellung in Figur 1 ist - das gleiche gilt auch für die Darstellungen in den Figuren 2 und 3 -, um eine deutlichere Darstellung zu erreichen, ein im Vergleich zur Realität etwas aufgeweiteter Zustand des Systems aus Katheter und Stent 30 gezeigt. Gegenüber den Darstellungen in den Figuren 1 bis 3 sind die Flügel 12 des Ballons 10 eng an den Innenschaft 14 angelegt und der Stent 30 in der Realität eng auf den Ballon 10 des Katheters aufgecrimpt, so dass die äußeren Flächen 16 des Ballons 10 im nicht dilatierten Zustand durch das Grundgitter des Stents 30 fast vollständig bedeckt sind. Die pharmazeutisch aktive Substanz wird durch den aufgecrimpten Stent 30 vor Aus-/Abwaschung beispielsweise durch den Blutstrom, wenn das erfindungsgemäße System in einem Blutgefäß angewendet wird, geschützt.

In einem bevorzugten Ausführungsbeispiel enthält der Stent 30 ein biodegradierbares Material wie Magnesium, eine Magnesiumlegierung, Eisen, Wolfram, Zink und/oder deren Legierungen. Durch das Aufcrimpen des Stents 30 ist die pharmazeutisch aktive Substanz durch die geschlossene Anordnung geschützt. Außerdem wird die pharmazeutisch aktive Substanz bei der Ballondilatation direkt in ausreichender Menge an den Körperhohlraum, in den der Katheter eingeführt und der Stent 30 angeordnet wurde, abgegeben, wobei diese Peakload klinisch ausreichend wirksam ist. Weder beim Einführen noch nach dem Deflatieren und Zurückziehen des Katheters wird die pharmazeutisch aktive Substanz mechanisch abgerieben. Dies ist insbesondere dann gegeben, wenn bei in den Figuren nicht dargestellten, im Ballon vorgesehenen Faltelementen der Ballon 10 geordnet in den gefalteten Zustand vor dem Dilatieren zurückfindet und die pharmazeutisch aktive Substanz wieder zwischen den Falten geschützt ist.

In dem in Figur 2 dargestellten Ausführungsbeispiel ist die pharmazeutisch aktive Substanz auf oder in einem blättchenförmigen Träger 24 enthalten. Hierbei ist je ein solcher Träger 24 unter jedem Flügel 12 angeordnet. Dadurch kann bei einer mehrmaligen Verwendung des Katheters für das erfindungsgemäße System dieser mit einer erneuerten oder einer auf den entsprechenden Einsatzzweck und Patienten abgestimmten pharmazeutisch aktiven Substanz versehen werden. In weiteren Ausführungsbeispielen kann die pharmazeutisch aktive Substanz 20 auch, wie oben erläutert, mittels eines flüssigen Trägers, der beispielsweise ein Lösungsmittel oder ein Polymer enthält, aufgebracht werden.

In dem anhand von Figur 3 dargestellten Ausführungsbeispiel ist der Stent 30 gegenüber dem ersten, anhand von Figur 1 dargestellten Ausführungsbeispiel zusätzlich luminal mit einer leicht abwaschbaren Beschichtung 32 versehen, bestehend vorzugsweise aus einer oder mehreren der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle.

Ein erfindungsgemäßes System könnte mittels des folgenden Prozesses hergestellt werden:

Zunächst wird der Ballon 10 des Katheters aufgeblasen und anschließend unter leichtem Überdruck mit einer oder mehreren pharmazeutisch aktiven Substanzen 20 beschichtet (beispielsweise mittels Tauchen oder Sprühen). Die distalen und proximalen Enden des Ballons und die Ballonkonen können hierbei abgedeckt werden, so dass diese keine Beschichtung durch pharmazeutisch aktive Substanz 20 erhalten. Anschließend wird der Ballon 10 gefaltet, d.h. mit Flügeln versehen und die Flügel an den Innenschaft 14 angelegt, wobei zum Schutz der Beschichtung eine Folie zwischen Ballonfaltvorrichtung und Ballon angeordnet werden kann. In einem nächsten Schritt wird pharmazeutisch aktive Substanz 20 entfernt, die auf den äußeren Flächen 16 der Flügel 12, die nach dem Falten und Anlegen des Ballons 10 die Oberfläche des gefalteten Ballons bilden, angeordnet ist, beispielsweise mittels Wegwischen, zum Beispiel mit porösem Zellpapier, Schwamm oder Ähnlichem, gegebenenfalls getränkt mit einem Lösungsmittel. Anschließend kann die pharmazeutisch aktive Substanz 20 ausgehärtet oder polymerisiert werden, beispielsweise mittels UV-Bestrahlung, Betastrahlung und/oder einer thermischen Behandlung. Anschließend wird der Stent 30 auf den gefalteten und mit pharmazeutisch aktiver Substanz 20 versehenen Ballon 10 aufgecrimpt.

Alternativ zu der Überdruck-Beschichtung mit der pharmazeutisch aktiven Substanz kann auch ein wirkstoffbeladener, gefalteter blättchenförmiger Träger 24 in der Falz, d.h. unter dem Flügel 12, eingebracht werden und dort unter Druckeinwirkung verklebt werden.

Bei einem weiteren alternativen Herstellungsverfahren kann ein blättchenförmiger Träger mit einem Gemisch aus der pharmazeutisch aktiven Substanz und einem Lösemittel unter einen Flügel 12 geklemmt und anschließend unter äußerer Druckeinwirkung herausgezogen werden, so dass ein Großteil der pharmazeutisch aktiven Substanz abgestreift wird und unter dem Flügel 12 verbleibt.

### Bezugszeichenliste

- 10: Ballon
- 12: Flügel
- 14: Innenschaft
- 16: äußere Fläche des Flügels 12
- 20: pharmazeutisch aktive Substanz(en)
- 24: Träger
- 30: Stent
- 32: Beschichtung

## Patentansprüche

1. System zum Einbringen einer intraluminalen Endoprothese (30), vorzugsweise eines Stents, in einen Körperhohlraum bestehend aus der intraluminalen Endoprothese (30) und einem Katheter mit einem Ballon (10), wobei der Ballon (10) in einem nicht dilatierten Zustand mindestens einen Flügel (12) aufweist, und wobei zumindest teilweise unter dem mindestens einen Flügel (12) des Ballons (10) im nicht dilatierten Zustand mindestens eine pharmazeutisch aktive Substanz (20) angeordnet ist und die intraluminale Endoprothese (30) auf dem gefalteten Ballon (10) derart fest angeordnet ist, dass sie diesen mindestens teilweise umgibt, **dadurch** gekannzeichnet, dass der mindestens eine Flügel (12) des Ballons (10) mittels der mindestens einen pharmazeutisch aktiven Substanz (20) verklebt ist.

2. System nach Anspruch 1, **dadurch gekennzeichnet, dass** die intraluminale Endoprothese (30) auf den Ballon (10) aufgecrimpt ist und/oder dass die intraluminalen Endoprothese (30) als biodegradierbarerer Stent, vorzugsweise als absorbierbarer Metallstent, ausgebildet ist.

3. System nach einem der vorhergehenden Ansprüche, **dadurch gekennzeichnet, dass** mindestens eine pharmazeutisch aktive Substanz (20) auf oder in einem Träger (24) oder mehreren Trägern angeordnet ist, die unter einem Flügel oder mehreren Flügeln (12) des Ballons (10) angeordnet sind.

4. System nach einem der vorhergehenden Ansprüche, **dadurch gekennzeichnet, dass** die intraluminale Endoprothese (30) die unter der Endoprothese (30) liegenden äußeren Flächen (16) des gefalteten Ballons (10) im nicht dilatierten Zustand bedeckt und/oder dass die intraluminale Endoprothese (30) luminal mit einer leicht lösbaren Beschichtung (32) versehen ist, vorzugsweise mit einer oder mehreren der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle.

5. System nach einem der vorhergehenden Ansprüche, **dadurch gekennzeichnet, dass** die mindestens eine pharmazeutisch aktive Substanz (20) mittels Tauchen, Sprühen, Bestreichen oder Pressen auf dem Ballon (10) aufgebracht ist und/oder dass die mindestens eine pharmazeutisch aktive Substanz (20) Taxole und/oder Taxane, vorzugsweise Paclitaxel und/oder Sirolimus, und/oder einen hyperplastischen Wirkstoff mit einem Verteilungskoeffizienten zwischen Butanol und Wasser von ≥ 0,5 enthält.

6. System nach einem der vorhergehenden Ansprüche, **dadurch gekennzeichnet, dass** der Ballon (10) mindestens ein im Wesentlichen in Längsrichtung verlaufendes Faltelement vorsieht, das beim Falten des Ballons (10) in einem der in Längsrichtung verlaufenden Bereiche des Flügels (12) mit einem Minimum im Biegeradius angeordnet ist und/oder dass der Ballon (10) entlang des mindestens einen Faltelements einen Bereich des Ballons (10) ausbildet, der verglichen mit den übrigen Bereichen des Ballons (10) eine veränderte Steifigkeit, vorzugsweise eine niedrigere Steifigkeit, aufweist und/oder dass der Ballon (10) in dem Bereich des mindestens einen Faltelements Vertiefungen oder Erhebungen oder mindestens einen Wandstärkensprung aufweist.

7. System nach Anspruch 6, **dadurch gekennzeichnet, dass** das mindestens eine Faltelement einen Bereich des Ballons (10) ausbildet, der eine von den übrigen Bereichen des Ballons (10) verschiedene Materialzusammensetzung aufweist und/oder dass das mindestens eine Faltelement Unterbrechungen aufweist und/oder dass das mindestens eine Faltelement in einem fest vorgegebenen Winkel zur Ballonachse verläuft und/oder dass die Faltelemente des Ballons (10) durch Längsstreben ausgebildet werden, die ein Gerüst bilden, welches an der Innenseite und/oder an der Außenseite des Ballons angeordnet ist und das den Ballon an definierten Stellen stützt.

8. Verfahren zur Herstellung eines Systems nach einem der vorhergehenden Ansprüche bestehend aus einem Katheter mit einem Ballon (10) und einer intraluminalen Endoprothese (30), **dadurch gekennzeichnet, dass** zunächst mindestens eine pharmazeutisch aktive Substanz (20), ggf. eingebettet in einen Träger, auf oder in die äußere Oberfläche des Ballons (10) auf- oder eingebracht wird, vorzugsweise mittels Tauchen, Sprühen, Bestreichen oder Pressen, anschließend der Ballon (10) mit mindestens einem Flügel (12) versehen, dann dieser mindestens eine Flügel (12) eng angelegt und danach die intraluminale Endoprothese (30) auf dem gefalteten Ballon (10) derart fest angeordnet wird, dass sie diesen mindestens teilweise umgibt.

9. Verfahren nach Anspruch 8, **dadurch gekennzeichnet, dass** die intraluminale Endoprothese (30) mittels Aufcrimpen auf dem Ballon (10) angeordnet wird und/oder dass die distalen und proximalen Enden des Ballons (10) beim Aufbringen der mindestens einen pharmazeutisch aktiven Substanz ausgespart werden, vorzugsweise durch Abdecken während des Aufbringens der Substanz.

10. Verfahren nach Anspruch 8 oder 9, **dadurch gekennzeichnet, dass** die überschüssige mindestens eine pharmazeutisch aktive Substanz (20) auf den nach dem Falten außen liegenden Oberflächen (16) des Ballons (10) nach dem Faltschritt entfernt werden, vorzugsweise durch Wegwischen und/oder dass die mindestens eine pharmazeutisch aktive Substanz (20), ggf. mit dem Träger, vor der Anordnung der intraluminalen Endoprothese (30) auf dem Ballon (10) ausgehärtet oder polymerisiert wird und/oder dass die mindestens eine pharmazeutisch aktive Substanz (20) vor der Anordnung der intraluminalen Endoprothese (30) auf dem Ballon (10) mittels eines Lösungsmittels als Träger, vorzugsweise DMSO, Aceton, Ether (Di-ethylether), Methanol, Isopropanol oder Ester, in Lösung aufgetragen wird, wobei das Lösungsmittel beim oder nach dem Auftragen verdampft und/oder dass die mindestens eine pharmazeutisch aktive Substanz (20) mittels eines Polymers oder einer polymerähnlichen Substanz als Träger, vorzugsweise Hyaloronsäure, P4HB, Polyvinylpyrolidon, Liposome, Nanopartikel, Seidenproteine oder Cyclodextrine, aufgetragen und anschließend ausgehärtet wird und/oder dass die pharmazeutisch aktive Substanz (20) dann auf dem Ballon (10) aufgebracht wird, wenn sich der Ballon (10) im dilatierten Zustand befindet.

11. Verfahren nach einem der Ansprüche 8 bis 10, **dadurch gekennzeichnet, dass** die intraluminale Endoprothese (30) vor der Anordnung der intraluminalen Endoprothese (30) auf dem Ballon (10) luminal mit einer leicht abwaschbaren Beschichtung (32), vorzugsweise mit einer oder mehrerer der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle, versehen wird.

12. Verfahren nach einem der Ansprüche 8 bis 11, **dadurch gekennzeichnet, dass** der Ballon (10) vor oder nach der Verbindung mit den übrigen Teilen des Katheters und vor dem ersten Falten mit mindestens einem Faltelement versehen wird und/oder dass der Ballon (10) vor der Verbindung mit den übrigen Teilen des Katheters mittels Blasformen hergestellt wird, wobei die Blasform dort, wo das mindestens eine Faltelement entstehen soll, mit einer Vertiefung oder einer Erhebung versehen ist und/oder dass der Ballon (10) vor der Verbindung mit den übrigen Teilen des Katheters mittels Blasformen oder Spritzblasen derart hergestellt wird, dass im Bereich des mindestens einen Faltelements mindestens ein definierter Wandstärkensprung erzeugt wird und/oder dass der Ballon (10) außen und/oder innen mit einem Längsstreben als Faltelemente enthaltenden Gerüst verbunden wird, welches den Ballon (10) in bestimmten Bereichen stützt und/oder dass der Ballon (10) im Bereich des mindestens einen Faltelements, vorzugsweise vor dem Aufbringen der pharmazeutisch aktiven Substanz (20), lokal thermisch behandelt wird und/oder dass der Ballon (10), vorzugsweise vor dem Aufbringen der pharmazeutisch aktiven Substanz (20), im Bereich des mindestens einen Faltelements mittels eines Lösungsmittels behandelt wird und/oder dass der Ballon (10), vorzugsweise vor dem Aufbringen der pharmazeutisch aktiven Substanz (20), im Bereich außerhalb des mindestens einen Faltelements mit einem weiteren verstärkenden Material versehen wird.

13. Verfahren nach Anspruch 12, **dadurch gekennzeichnet, dass** das mindestens eine Faltelement derart vorgesehen wird, dass der Flügel unter einem fest vorgegebenen Winkel zur Ballonachse verläuft.

14. Verfahren nach einem der Ansprüche 8 bis 13, **dadurch gekennzeichnet, dass** der Innenschaft und der Außenschaft des Katheters vor dem Verbinden miteinander verdreht und/oder verschoben werden, wobei der Ballon (10) vor dem Verdrehen und/oder Verschieben bereits mit Innenschaft und Außenschaft verbunden ist.

## Claims

1. A system for introducing an intraluminal endoprosthesis (30), preferably a stent, into a body cavity, the system consisting of the intraluminal endoprosthesis (30) and a catheter with a balloon (10), wherein in a non-dilated state, the balloon (10) has at least one wing (12), and wherein at least one pharmaceutically active substance (20) is arranged at least partially underneath the at least one wing (12) of the balloon (10) in the non-dilated state, and the intraluminal endoprosthesis (30) is fixedly arranged on the folded balloon (10) in such a manner that the intraluminal endoprosthesis at least partially encloses the balloon, **characterized in that** the at least one wing (12) of the balloon (10) is adhesively bonded by means of the at least one pharmaceutically active substance (20).

2. The system according to claim 1, **characterized in that** the intraluminal endoprosthesis (30) is crimped onto the balloon (10) and/or that the intraluminal endoprosthesis (30) is configured as biodegradable stent, preferably as absorbable metal stent.

3. The system according to any one of the preceding claims, **characterized in that** at least one pharmaceutically active substance (20) is arranged on or in a carrier (24) or a plurality of carriers which are arranged underneath a wing or a plurality of wings (12) of the balloon (10).

4. The system according to any one of the preceding claims, **characterized in that** the intraluminal endoprosthesis (30) covers the outer faces (16) of the folded balloon (10) which are underneath the endoprosthesis in the non-dilated state, and that the intraluminal endoprosthesis (30) is provided luminally with an easily dissolvable coating (32), preferably with one or more of the substances from the group sugars, preferably polysaccharides, glycans, glucose, glycogen, amylase, amylopectin, chitin, callose and cellulose, and fats, preferably cholesterol, palm oil, partially hydrogenated soy oils and saturated oils.

5. The system according to any one of the preceding claims, **characterized in that** the at least one pharmaceutically active substance (20) is applied to the balloon (10) by means of immersion, spraying, brushing or pressing, and/or that the at least one pharmaceutically active substance (20) contains taxols and/or taxanes, preferably paclitaxel and/or sirolimus, and/or a hyperplastic active ingredient having a distribution coefficient between butanol and water of ≥ 0.5.

6. The system according to any one of the preceding claims, **characterized in that** the balloon (10) provides at least one folding element which runs substantially in the longitudinal direction and which, when folding the balloon (10), is arranged with a minimum in the bending radius in one of the areas of the wing (12) running substantially in the longitudinal direction, and/or that along the at least one folding element, the balloon (10) forms an area of the balloon which area, in comparison with the rest of the areas of the balloon (10), has a changed stiffness, preferably a lower stiffness, and/or that the balloon (10) has recesses or elevations or at least one sudden change in wall thickness in the area of the at least one folding element.

7. The system according to claim 6, **characterized in that** the at least one folding element forms an area of the balloon (10) which has a material composition that is different from the rest of the areas of the balloon (10), and/or that the at least one folding element has interruptions, and/or that the at least one folding element runs at a fixed predefined angle to the balloon axis, and/or that the folding elements of the balloon (10) are formed by longitudinal struts which form a scaffold that is arranged on the inside or outside of the balloon and which supports the balloon at defined locations.

8. A method for manufacturing a system according to any one of the preceding claims, consisting of a catheter with a balloon (10) and an intraluminal endoprosthesis (30), **characterized in that** first at least one pharmaceutically active ingredient (20), if necessary embedded in a carrier, is applied to or introduced in the outer surface of the balloon (10), preferably by means of immersion, spraying, brushing or pressing, subsequently, the balloon (10) is provided with at least one wing (12), then said at least one wing (12) is arranged in a close contact, and after this, the intraluminal endoprosthesis (30) is fixedly arranged on the folded balloon in such a manner that it at least partially encloses said balloon.

9. The method according to claim 8, **characterized in that** the intraluminal endoprosthesis (30) is arranged on the balloon by means of crimping, and/or that the distal and proximal ends of the balloon (10) remain uncoated when applying the at least one pharmaceutically active substance, preferably by covering the ends when applying the substance.

10. The method according to claim 8 or claim 9, **characterized in that** the excess of the at least one pharmaceutically active substance (20) on the surfaces (16) of the balloon (10) which are on the outside after folding is removed after the folding step, preferably by wiping off, and/or that the at least one pharmaceutically active substance (20), if necessary with the carrier, is cured or polymerized before arranging the intraluminal endoprosthesis (30) on the balloon (10), and/or that the at least one pharmaceutically active substance (20) is applied in solution on the balloon (10) by means of a solvent as carrier, preferably DMSO, acetone, ether (diethyl-ether), methanol, isopropanol or ester before arranging the intraluminal endoprosthesis on the balloon (10), wherein the solvent evaporates during or after the application, and/or that the at least one pharmaceutically active substance (20) is applied by means of a polymer or polymer-like substance as the carrier, preferably hyaloronic acid, P4HB, polyvinylpyrrolidone, liposomes, nano-particles, silk proteins or cyclodextrines, and is subsequently cured, and/or that the pharmaceutically active substance (20) is applied to the balloon (10) when the balloon is in the dilated state.

11. The method according to any one of the claims 8 to 10, **characterized in that** before arranging the intraluminal endoprosthesis (30) on the balloon (10), the intraluminal endoprosthesis (30) is luminally coated with a coating (32) that can be easily washed off, preferably with one or more of the substances from the group sugars, preferably polysaccharides, glycans, glucose, glycogen, amylase, amylopectin, chitin, callose and cellulose, and fats, preferably cholesterol, palm oil, partially hydrogenated soy oils and saturated oils.

12. The method according to any one of the claims 8 to 11, **characterized in that** before or after connecting to the rest of the parts of the catheter and before the first folding, the balloon (10) is provided with at least one folding element, and/or that before connecting to the other parts of the catheter, the balloon (10) is manufactured by means of blow molding, wherein at the location where the at least one folding element is to be formed, the blow mold is provided with a recess or an elevation, and/or that before connecting to the rest of the parts of the catheter, the balloon (10) is manufactured by means of blow molding or injection blow molding in such a manner that in the area of the at least one folding element, at least one defined sudden change in wall thickness is generated, and/or that the balloon (10) is connected on the outside and/or inside to a scaffold containing longitudinal struts as folding elements, the scaffold supporting the balloon (10) in certain areas, and/or that preferably before applying the pharmaceutically active substance (20), the balloon (10) is treated locally in a thermal manner, and/or that preferably before applying the pharmaceutically active substance (20), the balloon (10) is treated by means of a solvent in the area of the at least one folding element, and/or that preferably before applying the pharmaceutically active substance (20), the balloon (10) is provided with a further reinforcing material in the area outside of the at least one folding element.

13. The method according to claim 12, **characterized in that** the at least one folding element is provided in such a manner that the wing runs at a fixed predefined angle to the balloon axis.

14. The method according to any one of the claims 8 to 13, **characterized in that** the inner shaft and the outer shaft of the catheter are rotated or displaced before joining the inner shaft and the outer shaft together, wherein the balloon (10) is already connected to the inner shaft and the outer shaft before being rotated or displaced.

## Revendications

1. Système pour l'insertion d'une endoprothèse intraluminale (30), de préférence d'un stent, dans un espace creux d'un corps constitué de l'endoprothèse intraluminale (30) et d'un cathéter avec un ballon (10), dans lequel le ballon (10), dans un état non dilaté, comporte au moins une aile (12) et dans lequel au moins une substance active pharmaceutique (20) est placée au moins partiellement sous l'au moins une aile (12) du ballon (10), à l'état non dilaté, et l'endoprothèse intraluminale (30) est placée serrée contre le ballon plié (10), de manière à l'entourer au moins partiellement, **caractérisé en ce que** l'au moins une aile (12) du ballon (10) est encollée au moyen de l'au moins une substance active pharmaceutique (20).

2. Système selon la revendication 1, **caractérisé en ce que** l'endoprothèse intraluminale (30) est sertie sur le ballon (10) et/ou **en ce que** l'endoprothèse intraluminale (30) est formée comme un stent biodégradable, de préférence comme un stent métallique absorbable.

3. Système selon l'une des revendications précédentes, **caractérisé en ce qu'**au moins une substance active pharmaceutique (20) est placée sur ou dans un support (24) ou sur ou dans plusieurs supports disposés sous une aile ou sous plusieurs ailes (12) du ballon (10).

4. Système selon l'une des revendications précédentes, **caractérisé en ce qu'**à l'état non dilaté, l'endoprothèse intraluminale (30) recouvre les surfaces extérieures (16) du ballon plié (10) qui sont situées sous l'endoprothèse (30), et/ou **en ce que** l'endoprothèse intraluminale (30) est pourvu de façon luminale d'un revêtement (32) facilement retirable, de préférence de l'une ou plusieurs des substances parmi le groupe comprenant le sucre, de préférence le polysaccaride, le glycane, le glucose, le glycogène, l'amylose, l'amylopectine, la chitine, la callose et la cellulose, et des graisses, de préférence la cholestérine, le cholestérol, l'huile de palme, les huiles de soja partiellement hydrogénées et les huiles saturées.

5. Système selon l'une des revendications précédentes, **caractérisé en ce que** l'au moins une substance active pharmaceutique (20) est appliqué sur le ballon (10) au moyen d'une immersion, d'une pulvérisation, d'un badigeonnage ou d'un pressage, et/ou **en ce que** l'au moins une substance active pharmaceutique (20) contient des taxols et/ou des taxanes, de préférence du Paclitaxel et/ou du Sirolimus, et/ou une substance active hyperplastique avec un coefficient de répartition entre le butanol et l'eau, de ≥ 0,5.

6. Système selon l'une des revendications précédentes, **caractérisé en ce que** le ballon (10) prévoit au moins un élément à pli, s'étendant essentiellement dans le sens longitudinal, qui, lors du pliage du ballon (10), se trouve dans l'une des régions de l'aile (12) s'étendant dans le sens longitudinal, avec un minimum dans le rayon de courbure, et/ou **en ce que** le long de l'au moins un élément à pli, le ballon (10) forme une région du ballon (10) présentant une rigidité différente par rapport aux autres régions du ballon (10), de préférence une rigidité inférieure, et/ou **en ce que** dans la région de l'au moins un élément à pli, le ballon (10) comporte des renfoncements ou des surélévations ou au moins une différence d'épaisseur de paroi.

7. Système selon la revendication 6, **caractérisé en ce que** l'au moins un élément à pli forme une région du ballon (10) présentant une composition de matériau différente des autres régions du ballon (10), et/ou **en ce que** l'au moins un élément à pli comporte des interruptions, et/ou **en ce que** l'au moins un élément à pli s'étend en formant un angle fixement prédéfini par rapport à l'axe de ballon, et/ou **en ce que** les éléments à pli du ballon (10) sont formés par des nervures longitudinales formant une structure placée du côté intérieur et/ou du côté extérieur du ballon et soutenant le ballon à des endroits prédéfinis.

8. Procédé pour la fabrication d'un système selon l'une des revendications précédentes, constitué d'un cathéter avec un ballon (10) et d'une endoprothèse intraluminale (30), **caractérisé en ce qu'**au moins une substance active pharmaceutique (20), éventuellement enrobée dans un support, est tout d'abord appliquée sur ou intégrée dans la surface extérieure du ballon (10), de préférence au moyen d'une immersion, d'une pulvérisation, d'un badigeonnage ou d'un pressage, puis le ballon (10) est pourvu d'au moins une aile (12), puis cette au moins une aile (12) est appliquée de façon serrée, et l'endoprothèse intraluminale (30) est ensuite placée fixement sur le ballon plié (10), de manière à l'entourer au moins partiellement.

9. Procédé selon la revendication 8, **caractérisé en ce que** l'endoprothèse intraluminale (30) est placée sur le ballon (10) au moyen d'un sertissage, et/ou en ce que lors de l'application de l'au moins une substance active pharmaceutique (20), les extrémités distales et proximales du ballon (10) sont épargnées, de préférence en les recouvrant pendant l'application de la substance.

10. Procédé selon la revendication 8 ou 9, **caractérisé en ce que** l'excédent de l'au moins une substance active pharmaceutique (20) sur les surfaces (16) du ballon (10) situées à l'extérieur après le pliage est retiré après l'étape de pliage, de préférence par essuyage, et/ou **en ce que** l'au moins une substance active pharmaceutique (20), éventuellement pourvue d'un support, est durcie ou polymérisée avant la disposition de l'endoprothèse intraluminale (30) sur le ballon (10), et/ou **en ce qu'**avant la disposition de l'endoprothèse intraluminale (30) sur le ballon (10), l'au moins une substance active pharmaceutique (20) est appliquée en solution à l'aide d'un solvant comme support, de préférence du DMSO, de l'acétone, de l'éther (diéthyle éther), du méthanol, de l'isopropanol ou de l'ester, le solvant s'évaporant pendant ou après l'application, et/ou **en ce que** l'au moins une substance active pharmaceutique (20) est appliquée à l'aide d'un polymère ou d'une substance semblable à un polymère comme support, de préférence de l'acide hyaluronique, du P4HB, du polyvinyle pyrrolidone, des liposomes, des nanoparticules, des protéines de soie ou des cyclodextrines, puis durcie, et/ou **en ce que** la substance active pharmaceutique (20) est appliquée sur le ballon (10) lorsque le ballon (10) est dans l'état dilaté.

11. Procédé selon l'une des revendications 8 à 10, **caractérisé en ce qu'**avant la disposition de l'endoprothèse intraluminale (30) sur le ballon (10), l'endoprothèse intraluminale (30) est pourvue de façon luminale d'un revêtement (32) facilement lavable, de préférence d'une ou plusieurs des substances parmi le groupe comprenant le sucre, de préférence le polysaccaride, le glycane, le glucose, le glycogène, l'amylose, l'amylopectine, la chitine, la callose et la cellulose, et des graisses, de préférence la cholestérine, le cholestérol, l'huile de palme, les huiles de soja partiellement hydrogénées et les huiles saturées.

12. Procédé selon l'une des revendications 8 à 11, **caractérisé en ce qu'**avant ou après la liaison avec les autres pièces du cathéter et avant le premier pliage, le ballon (10) est pourvu d'au moins un élément à pli, et/ou **en ce qu'**avant ou après la liaison avec les autres pièces du cathéter, le ballon (10) est réalisé au moyen d'un moulage par soufflage, le moule de soufflage étant pourvu d'un renfoncement ou d'une surélévation à l'endroit où l'au moins un élément à pli est censé se former, et/ou **en ce qu'**avant ou après la liaison avec les autres pièces du cathéter, le ballon (10) est réalisé de telle manière au moyen d'un moulage par soufflage ou d'un gonflage par injection, que dans la région de l'au moins un élément à pli est produite au moins une différence d'épaisseur de paroi prédéfinie, et/ou **en ce que** le ballon (10) est relié, à l'extérieur et/ou à l'intérieur, à une structure contenant des nervures longitudinales comme éléments à pli, laquelle soutient le ballon (10) dans des régions prédéfinies, et/ou **en ce que** dans la région de l'au moins un élément à pli, de préférence avant l'application de la substance active pharmaceutique (20), le ballon (10) est traité localement de façon thermique, et/ou **en ce que** de préférence avant l'application de la substance active pharmaceutique (20), le ballon (10) est traité au moyen d'un solvant dans la région de l'au moins un élément à pli, et/ou **en ce que** de préférence avant l'application de la substance active pharmaceutique (20), le ballon est pourvu d'un autre matériau renforçant dans la région à l'extérieur de l'au moins un élément à pli.

13. Procédé selon la revendication 12, **caractérisé en ce que** l'au moins un élément à pli est prévu de telle manière que l'aile s'étend sous un angle fixement prédéfini par rapport à l'axe de ballon.

14. Procédé selon l'une des revendications 8 à 13, **caractérisé en ce que** la tige intérieure et la tige extérieure du cathéter sont tournées et/ou décalées, avant d'être reliées entre elles, le ballon (10) étant déjà relié à la tige intérieure et à la tige extérieure avant la rotation et/ou le décalage.
